## Europäisches Patentamt
## European Patent Office
## Office européen des brevets

(19)

(11) Numéro de publication: **0 295 166**
**A1**

(12)

# DEMANDE DE BREVET EUROPEEN

(21) Numéro de dépôt: 88401261.8

(22) Date de dépôt: 24.05.88

(51) Int. Cl.⁴: **A 61 K 31/195**
A 61 K 31/415
//(A61K31/415,31:405,31:195,
31:19)

(30) Priorité: 27.05.87 FR 8707459

(43) Date de publication de la demande:
14.12.88 Bulletin 88/50

(84) Etats contractants désignés:
AT BE CH DE FR GB IT LI LU NL SE

(71) Demandeur: SYNTHELABO
58 rue de la Glacière
F-75013 Paris (FR)

(72) Inventeur: Bobee, Jean-Marc
Résidence "Le Village" 3, Square des Poètes
F-91370 Verrières le Buisson (FR)

Melin, Christian
1, Square des Ecrivains
F-91370 Verrières le Buisson (FR)

(74) Mandataire: Thouret-Lemaitre, Elisabeth et al
Service Brevets - SYNTHELABO 58, rue de la Glacière
F-75013 Paris (FR)

(54) Compositions pharmaceutiques contenant un mélange de sels de cétoacides et d'amino-acides.

(57) Compositions pharmaceutiques contenant un mélange de sels formés par des α-céto-acides et des acides aminés basiques et de la L-tyrosine, de la L-thréonine, du L-tryptophane et du D,L-α-hydroxy-γ-méthyl-thio-butyrate de calcium.

EP 0 295 166 A1

Bundesdruckerei Berlin

**Description**

## COMPOSITIONS PHARMACEUTIQUES CONTENANT UN MELANGE DE SELS DE CETOACIDES ET D'AMINO-ACIDES

La présente invention a pour objet des compositions pharmaceutiques utiles pour le traitement de l'urémie.

Plus spécifiquement, l'invention a pour objet des compositions constituées par un mélange de sels formés par des $\alpha$-céto-analogues d'acides aminés essentiels à chaîne ramifiée et des acides aminés basiques

Les compositions de l'invention peuvent être utilisées en addition à un régime protidique et à un supplément en vitamines et ou éléments minéraux.

Les sels d'acides $\alpha$-aminés basiques et d'$\alpha$-céto-analogues d'acides aminés essentiels à chaîne ramifiée ont été décrits dans les brevets français 79.06533 (2419723) et 80.08318 (2454434).

Des compositions contenant un mélange de plusieurs de ces sels et pouvant contenir également de la L-tyrosine, de la L-thréonine et de l'$\alpha$-hydroxy-$\gamma$-méthylthiobutyrate de calcium ont été décrites dans la demande de brevet européen n° 86402806.3.

La présente invention a pour objet des compositions pharmaceutiques comprenant

1) un mélange de sels d'acides $\alpha$-aminés basiques et d'$\alpha$-céto-analogues d'acides aminés essentiels à chaîne ramifiée et 2) de la L-tyrosine, de la L-thréonine, du L-tryptophane et de l'$\alpha$-hydroxy-$\gamma$-méthylthiobutyrate de calcium.

Les sels d'acides $\alpha$-aminés basiques et d'$\alpha$-céto-analogues d'acides aminés essentiels à chaîne ramifiée sont choisis parmi les suivants :

- $\alpha$-céto-$\beta$-méthylvalérate de L-ornithine
- $\alpha$-céto-isovalérate de L-ornithine
- $\alpha$-céto-isocaproate de L-ornithine
- $\alpha$-céto-isocaproate de L-lysine
- $\alpha$-céto-isovalérate de L-lysine
- $\alpha$-céto-$\beta$-méthyl-valérate de L-lysine
- $\alpha$-céto-isocaproate de L-histidine.

En plus d'un mélange de sels, les compositions pharmaceutiques de l'invention contiennent de la L-tyrosine, de la L-thréonine, du L-tryptophane et du D,L-$\alpha$-hydroxy-$\gamma$-méthyl-thiobutyrate de calcium.

Les compositions pharmaceutiques de l'invention sont, de préférence, administrées avec un régime à taux faible en protéines et contenant de la vitamine B, de l'acide ascorbique et du calcium (sous forme de sel).

Les compositions de l'invention peuvent être constituées par un mélange de cinq sels ou de moins de cinq sels, selon les quantités de chacun des six amino-acides souhaitées.

Les compositions de l'invention contiennent tout ou partie des constituants suivants dans les proportions suivantes (% en poids du mélange total)

- $\alpha$-céto-isovalérate    5-20
(ou cétovaline)
- $\alpha$-céto-isocaproate    5-15
(ou cétoleucine
- $\alpha$-céto- $\beta$-méthyl-valérate    5-15
(ou cétoisoleucine)
- L-ornithine    15-25
- L-lysine    10-25
- L-histidine    1-10
- L-thréonine    1-10
- L-tyrosine    5-20
- L-tryptophane    0,1-5
- DL-$\alpha$-hydroxy-$\gamma$-méthylthio-butyrate de calcium.    1-5

Les exemples suivants illustrent l'invention :

## Exemple 1

(dose journalière administrée)

|  | g | % |
|---|---|---|
| cétovaline-ornithine ou α-ceto-isovalerate de L-ornithine | 1,738 | 9,26 |
| cétovaline-lysine ou α-ceto-isovalerate de L-lysine | 1,836 | 9,78 |
| cétoleucine-lysine ou α-céto-isocaproate de L-lysine | 3,869 | 20,62 |
| cétoleucine-histidine ou α-céto-isocaproate de L-histidine, $H_2O$ | 1,213 | 6,46 |
| cétoisoleucine-ornithine ou α-céto-β-méthylvalérate de L-ornithine | 3,672 | 19,57 |
| DL 2-hydroxy 4-(méthylthio) butyrate de calcium | 0,677 | 3,61 |
| L-thréonine | 1,786 | 9,52 |
| L-tyrosine | 3,624 | 19,31 |
| L-tryptophane | 0,350 | 1,87 |
|  | 18,765 | 100 |

## Exemple 2

|  | (dose journalière administrée) | |
|---|---|---|
|  | g | % |
| cétovaline-ornithine<br>ou α-ceto-isovalerate de L-ornithine | 3,342 | 17,53 |
| cétovaline-lysine<br>ou α-ceto-isovalerate de L-lysine | 3,634 | 19,06 |
| cétoleucine-lysine<br>ou α-céto-isovalerate de L-lysine | 3,563 | 18,69 |
| cétoleucine-histidine<br>ou α-céto-isocaproate de L-histidine | 1,300 | 6,82 |
| cétoisoleucine-ornithine<br>ou α-céto-β-méthylvalérate de L-ornithine | 3,000 | 15,73 |
| DL 2-hydroxy 4-(méthylthio) butyrate<br>de calcium | 0,677 | 3,55 |
| L-thréonine | 1,2 | 6,29 |
| L-tyrosine | 2 | 10,49 |
| L-tryptophane | 0,35 | 1,84 |
|  | 19,066 | 100 |

Exemple 3

(dose journalière administrée)

| | g | % |
|---|---|---|
| cétovaline-ornithine ou α-ceto-isovalerate de L-ornithine | 3,342 | 16,66 |
| cétovaline-lysine ou α-ceto-isocaproate de L-lysine | 3,634 | 18,11 |
| cétoleucine-lysine ou α-céto-isocaproate de L-lysine | 3,563 | 17,76 |
| cétoleucine-histidine ou α-céto-isocaproate de L-histidine | 1,300 | 6,48 |
| cétoisoleucine-ornithine ou α-céto-β-méthylvalérate de L-ornithine | 3,000 | 14,95 |
| DL 2-hydroxy 4-(méthylthio)-butyrate de calcium | 0,677 | 3,37 |
| L-thréonine | 1,700 | 8,47 |
| L-tyrosine | 2,500 | 12,46 |
| L-tryptophane | 0,35 | 1,74 |
| | 20,066 | 100 |

Les compositions pharmaceutiques de l'invention peuvent être administrées oralement ou par voie parentérale, elles sont cependant spécialement adaptées à la voie orale. Elles peuvent être présentées sous la forme de poudre, granulés ou comprimés.

Les compositions de l'invention peuvent contenir en plus des vitamines et des éléments minéraux, tels que l'acide ascorbique (vitamine C), un complexe de vitamines B, et du calcium, par exemple sous forme de carbonate ou de gluconate de calcium. De plus les compositions de l'invention sont administrées de préférence en addition à un régime protéinique de 20 à 30 mg/jour.

## Revendications

1. Compositions pharmaceutiques utiles pour le traitement de l'urémie, caractérisées par le fait qu'elles contiennent

1) un mélange d'au plus cinq des sels suivants :
- $\alpha$-céto-$\beta$-méthylvalérate de L-ornithine
- $\alpha$-céto-isovalérate de L-ornithine
- $\alpha$-céto-isocaproate de L-ornithine
- $\alpha$-céto-isocaproate de L-lysine
- $\alpha$-céto-isovalérate de L-lysine
- $\alpha$-céto-$\beta$-méthyl-valérate de L-lysine
- $\alpha$-céto-isocaproate de L-histidine ainsi que

2) de la L-tyrosine, de la L-thréonine, du L-tryptophane et du D,L-$\alpha$-hydroxy-$\gamma$-méthyl-thio-butyrate de calcium.

2. Compositions selon la revendication 1, caractérisées par le fait qu'elles contiennent tout ou partie des constituants suivants dans les proportions suivantes (% en poids du mélange total)
- $\alpha$-céto-isovalérate      5-20
(ou cétovaline)
- $\alpha$-céto-isocaproate      5-15
(ou cétoleucine)
- $\alpha$-céto-$\beta$-méthyl-valérate      5-15
(ou cétoisoleucine)
- L-ornithine      15-25
- L-lysine      10-25
- L-histidine      1-10
- L-thréonine      1-10
- L-tyrosine      5-20
- L-tryptophane      0,1-5
- DL-$\alpha$-hydroxy-$\gamma$-méthylthio-butyrate de calcium.      1-5

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X,Y | US-A-4 352 814  (MAcKENZIE WALSER) * Colonne 10, lignes 1-57, exemples II,III; colonne 12, lignes 25-46, revendications 5,6 * --- | 1-2 | A 61 K  31/195 A 61 K  31/415// (A 61 K  31/415 A 61 K  31:405 A 61 K  31:195 A 61 K  31:19 ) |
| D,Y | FR-A-2 419 723  (THE JOHNS HOPKINS UNIVERSITY) * Pages 17-18, revendications 1-18 * --- | 1-2 | |
| D,Y | FR-A-2 454 434  (THE JOHNS HOPKINS UNIVERSITY) * Page 21, lignes 19-35, revendications 18-20 * --- | 1-2 | |
| E,D | EP-A-0 227 545  (THE JOHN HOPKINS UNIVERSITY) * Page 19, ligne 1 - page 25, ligne 10, revendications 1-15 * ----- | 1-2 | |

**DOMAINES TECHNIQUES RECHERCHES (Int. Cl.4)**

A 61 K

Le présent rapport a été établi pour toutes les revendications

| Lieu de la recherche | Date d'achèvement de la recherche | Examinateur |
|---|---|---|
| LA HAYE | 08-09-1988 | BRINKMANN C. |